# EUROPEAN PATENT APPLICATION

(11) **EP 4 785 986 A1**
(43) Date of publication of application: **05.08.2026**
(21) Application number: 24923669.6
(22) Date of filing: 25.09.2024
(51) Int. Cl.: A61M 39/10, A61M 5/34

(54) **CONNECTION STRUCTURE OF MEDICAL INSTRUMENT AND SYRINGE ASSEMBLY**

(30) Priority: 08.02.2024 JP 2024017709
(71) Applicant: Terumo Kabushiki Kaisha, Tokyo 151-0072 (JP)
(72) Inventor: KITAORI, Yasunori, Nakakoma-gun, Yamanashi 409-3853 (JP); NAKAJIMA, Kentaro, Nakakoma-gun, Yamanashi 409-3853 (JP)
(74) Representative: Prüfer & Partner mbB Patentanwälte · Rechtsanwälte
(86) International application number: PCT/JP2024/034183
(87) International publication number: WO 2025/169535

(57) **Abstract**

A medical device (10) includes a connection structure (14) that connects a first medical device (121) having a first threaded portion (661) and a second medical device (122) having a second threaded portion (662) screwed to the first threaded portion (661). A first portion (S1) is provided where a first engagement surface (701A) of a first thread ridge (681) and a second engagement surface (702A) of a second thread ridge (682) come into contact with each other when the first threaded portion (661) and the second threaded portion (662) are screwed together. At a first portion (S1), a first angle (θ11) of the first engagement surface (701A) of the first thread ridge (681) is different from a second angle (θ21) of the second engagement surface (702A) of a second thread ridge (682).

## Description

### Technical Field

The present invention relates to a connection structure of a medical device for connecting a first medical device and a second medical device and a syringe assembly.

### Background Art

JP 2015-058198 A relates to a syringe filled with a medical fluid. The distal end of the syringe includes a nozzle portion capable of discharging a medical fluid.

### Citation List

### Patent Literature

Patent Literature 1: JP 2015-058198 A

### Summary of Invention

### Technical Problem

In the medical field, two medical devices may be used in a state of being connected to each other by screw engagement. For example, in a syringe, a form in which an outer cylinder and a needle are connected by screw engagement is known. In a structure in which the medical devices are connected by screw engagement as described above, it is desirable that the threaded portion of one medical device and the threaded portion of the other medical connector device are firmly connected.

### Solution to Problem

An object of the present invention is to solve the above-described problem.
(1) According to an aspect of the present invention, there is provided a connection structure of a medical device, the connection structure connecting a first medical device having a first threaded portion and a second medical device having a second threaded portion screwed to the first threaded portion, in which the first threaded portion of the first medical device is a tapered thread having a first thread ridge, the second threaded portion of the second medical device is a tapered thread having a second thread ridge that comes into contact with the first thread ridge of the first threaded portion, when the first threaded portion and the second threaded portion are screwed together, a first portion is provided where a first engagement surface of the first thread ridge and a second engagement surface of the second thread ridge come into contact with each other, the first engagement surface is inclined at a first angle with respect to a direction orthogonal to an axis of the first medical device, and the second engagement surface is inclined at a second angle with respect to a direction orthogonal to an axis of the second medical device, and at the first portion, the first angle of the first engagement surface of the first thread ridge is different from the second angle of the second engagement surface of the second thread ridge.

According to the connection structure of a medical device, when the first threaded portion and the second threaded portion are screwed together, the first engagement surface and the second engagement surface having mutually different angles do not come into surface contact with each other at the first portion. Therefore, when the first threaded portion and the second threaded portion are screwed together, the relative movement of the first threaded portion and the second threaded portion in the radial direction along the first and second engagement surfaces, which causes them to ride over each other, is effectively suppressed. Therefore, the first threaded portion and the second threaded portion can be firmly connected.

(2) In the connection structure of a medical device according to (1), the first medical device may be a syringe including a main body portion capable of being filled with a medical fluid, a nozzle portion provided at a distal end of the main body portion, and a lock portion surrounding the nozzle portion, the second medical device may be a puncture member including a connector having a hole portion capable of accommodating the nozzle portion and a puncture needle provided at a distal end of the connector, and the lock portion may include a first cylindrical portion having the first threaded portion on an inner peripheral surface, and the connector may include a second cylindrical portion having the second threaded portion on an outer peripheral surface.

In this configuration, by firmly connecting the first threaded portion of the first cylindrical portion and the second threaded portion of the second cylindrical portion, it is possible to effectively suppress leakage of the medical fluid when the medical device, in which the syringe and the puncture member are connected, administers the medical fluid.

(3) In the connection structure of a medical device according to (2), the first angle of the first engagement surface of the first threaded portion may be smaller than the second angle of the second engagement surface of the second threaded portion.

In this configuration, when the first threaded portion and the second threaded portion are screwed together, the first thread ridge of the first threaded portion bites into the second engagement surface of the second thread ridge of the second threaded portion, whereby the first medical device (syringe) and the second medical device (puncture member) can be firmly connected.

(4) In the connection structure of a medical device according to (2), the second angle of the second engagement surface of the second threaded portion may be smaller than the first angle of the first engagement surface of the first threaded portion.

In this configuration, when the first threaded portion and the second threaded portion are screwed together, the second thread ridge of the second threaded portion bites into the first engagement surface of the first thread ridge of the first threaded portion, whereby the first medical device (syringe) and the second medical device (puncture member) can be firmly connected.

(5) In the connection structure of a medical device according to (2), the puncture member may include an elastic body disposed in the hole portion of the connector, and pressed and compressed by a distal end of the nozzle portion when the syringe is connected.

In this configuration, when the syringe and the puncture member are connected, the elastic body is compressed by the distal end of the nozzle portion, whereby it is possible to effectively suppress leakage of the medical fluid through a space between the distal end of the syringe and the hole portion of the puncture member by the elastic body. Even when the syringe and the puncture member are biased in the direction of being separated from each other by the resilient force, the first thread ridge and the second thread ridge are effectively prevented from riding over each other in the radial direction.

(6) In the connection structure of a medical device according to (3), when the first threaded portion and the second threaded portion are screwed together, in a connection direction of the first medical device and the second medical device, a second portion may be provided on a side opposite to the first portion, and an angle of a first non-engagement surface of the first thread ridge at the second portion may be greater than the first angle of the first engagement surface of the first thread ridge at the first portion.

In this configuration, since the angle of the first non-engagement surface of the first thread ridge at the second portion can be set to be the same as the second angle of the second engagement surface of the second thread ridge, the first threaded portion can be easily formed at low cost as compared with a configuration in which both the first angle of the first engagement surface and the angle of the first non-engagement surface of the first thread ridge at the first and second portions are smaller than the second angle of the second engagement surface of the second thread ridge.

(7) In the connection structure of a medical device according to (4), when the first threaded portion and the second threaded portion are screwed together, in a connection direction of the first medical device and the second medical device, a second portion may be provided on a side opposite to the first portion, and an angle of a second non-engagement surface of the second thread ridge at the second portion may be greater than the second angle of the second engagement surface of the second thread ridge at the first portion.

In this configuration, since the angle of the second non-engagement surface of the second thread ridge at the second portion can be set to be the same as the first angle of the first engagement surface of the first thread ridge, the second threaded portion can be easily formed at low cost as compared with a configuration in which both the second angle of the second engagement surface and the angle of the second non-engagement surface of the second thread ridge at the first and second portions are smaller than the first angle of the first engagement surface of the first thread ridge.

(8) In the connection structure of a medical device according to any one of (1) to (7), a difference between the first angle of the first engagement surface of the first threaded portion and the second angle of the second engagement surface of the second threaded portion may be less than 25°.

In this configuration, the first medical device and the second medical device can be more firmly connected.

(9) A syringe assembly that has the connection structure of a medical device according to (2), in which the syringe assembly may include the syringe and the puncture member.

In this configuration, the syringe and the puncture member, which constitute the syringe assembly, can be firmly connected.

(10) A medical apparatus including the syringe assembly according to (9), the medical apparatus including: the syringe; the puncture member; and a case in which the puncture member is accommodated and held, in which in a state where the puncture member is held inside the case, the puncture member and the syringe may be capable of being connected by the first threaded portion and the second threaded portion, and when the syringe and the puncture member are connected, the puncture member may rotate relative to the case when a predetermined torque or more acts on the puncture member as the first threaded portion and the second threaded portion are screwed together.

In this configuration, even in a case where a predetermined torque or more to the extent that the puncture member and the case rotate relative to each other acts on the puncture member, the screw engagement between the first threaded portion and the second threaded portion is firm, such that the screw engagement between the first threaded portion and the second threaded portion is effectively maintained.

### Advantageous Effects of Invention

According to the present invention, when the first threaded portion of the first medical device and the second threaded portion of the second medical device are screwed together, the first engagement surface and the second engagement surface having mutually different angles do not come into surface contact with each other at the first portion. Therefore, when the first threaded portion and the second threaded portion are screwed together, the relative movement of the first threaded portion and the second threaded portion in the radial direction along the first and second engagement surfaces, which causes them to ride over each other, is effectively suppressed. Thus, the first threaded portion and the second threaded portion can be firmly connected.

### Brief Description of Drawings

[Fig. 1] Fig. 1 is an overall configuration diagram of a medical device according to an embodiment of the present invention.
[Fig. 2] Fig. 2 is an exploded perspective view of the medical device illustrated in Fig. 1.
[Fig. 3] Fig. 3 is an enlarged cross-sectional view of a distal portion of the medical device illustrated in Fig. 1.
[Fig. 4] Fig. 4 is an enlarged cross-sectional view of a connection structure in a medical device.
[Fig. 5] Fig. 5 is a cross-sectional view of a puncture needle package including a puncture member.
[Fig. 6] Fig. 6A is an enlarged cross-sectional view of a first threaded portion and a second threaded portion in Fig. 4. Fig. 6B is an enlarged cross-sectional view of a first threaded portion and a second threaded portion according to a modification example.
[Fig. 7] Fig. 7 is an explanatory diagram at the time of connecting a puncture member of a puncture needle package and a distal portion of a syringe.
[Fig. 8] Fig. 8 is a characteristic diagram illustrating a relationship between a first angle of a first engagement surface of a first threaded portion and a torque applied when connecting a first medical device and a second medical device.
[Fig. 9] Fig. 9 is an explanatory diagram illustrating a state of administering a medical fluid when a puncture needle of a medical device has punctured a patient.

### Description of Embodiments

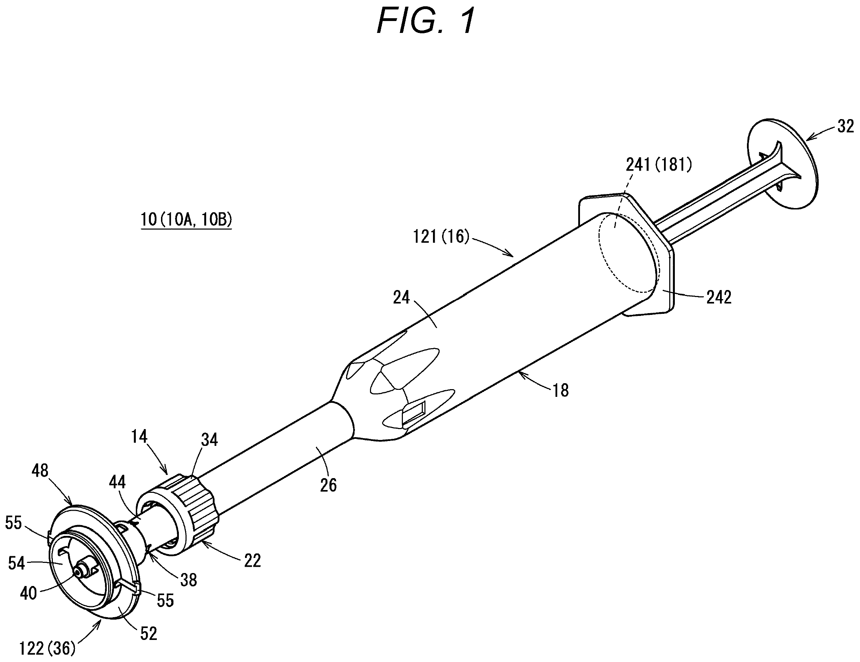

As illustrated in Fig. 1, a medical device 10 according to the present embodiment includes a first medical device 121, a second medical device 122, and a connection structure 14 that connects the first medical device 121 and the second medical device 122.

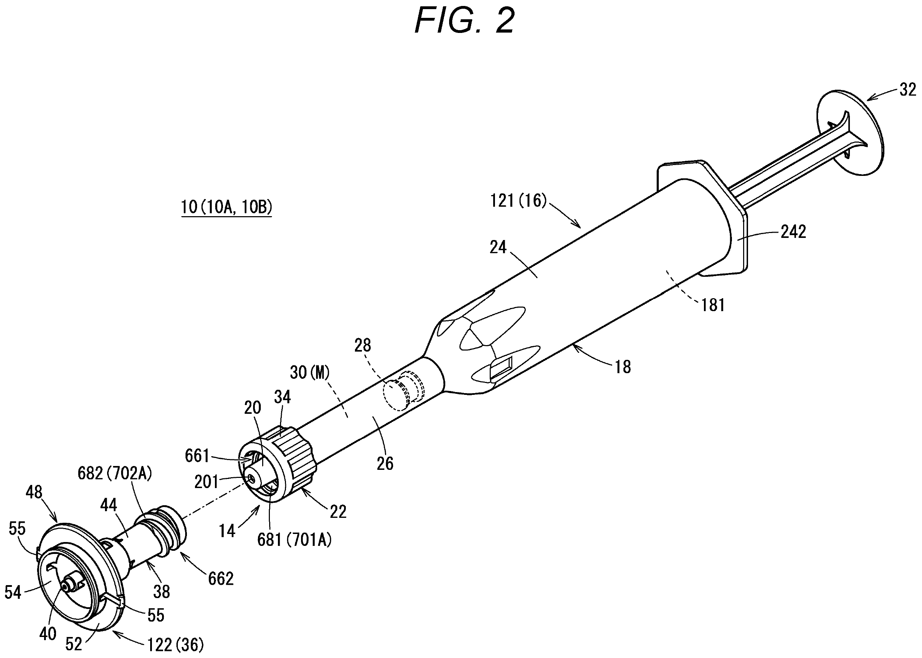

As illustrated in Fig. 2, the first medical device 121 is a syringe 16. The syringe 16 is, for example, a prefilled syringe. The syringe 16 includes a main body portion 18 that can be filled with a medical fluid M, a nozzle portion 20 provided at the distal end of the main body portion 18, and a lock portion 22 surrounding the nozzle portion 20.

The main body portion 18 is formed in a cylindrical shape having a lumen 181 therein. The main body portion 18 is formed of a transparent resin material or glass through which the lumen 181 is visible. The main body portion 18 includes a barrel portion 24 and a neck portion 26 provided at the distal end of the barrel portion 24. The proximal end of the barrel portion 24 includes a proximal end opening portion 241 in which the lumen 181 opens and a flange 242 protruding radially outward.

The neck portion 26 is formed to be smaller in diameter than the barrel portion 24. The neck portion 26 protrudes in a distal direction from the distal end of the barrel portion 24. A gasket 28 is inserted into the lumen 181 of the neck portion 26 through the proximal end opening portion 241.

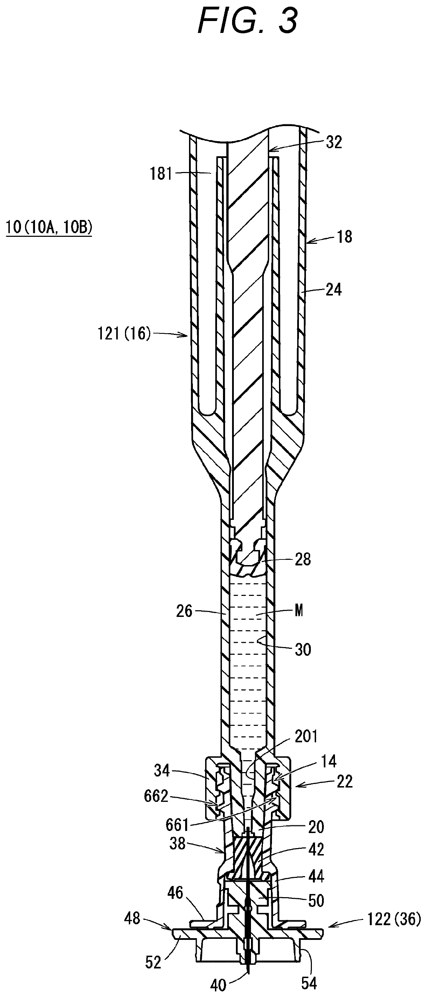

As illustrated in Fig. 3, the gasket 28 is formed of an elastic material and is axially slidably accommodated in the lumen 181. The gasket 28 liquid-tightly seals the inside of the lumen 181. A portion of the lumen 181 located on the distal side of the gasket 28 is a drug chamber 30 filled with the medical fluid M. The drug chamber 30 is filled with, for example, a liquid preparation such as a medical fluid M, injection water for dilution, or physiological saline in advance. The gasket 28 is provided at the distal end of a plunger 32 inserted into the lumen 181 of the barrel portion 24. The gasket 28 is slidably operated along the barrel portion 24 by the plunger 32. A proximal portion of the plunger 32 is exposed in a proximal direction from the proximal end of the main body portion 18 (see Fig. 1).

The nozzle portion 20 is provided at the distal end of the neck portion 26. The nozzle portion 20 is much smaller in diameter than the neck portion 26. The outer peripheral surface of the nozzle portion 20 is a Luer taper extending in the distal direction. The nozzle portion 20 protrudes in the distal direction from the distal end of the neck portion 26. A flow passage 201 is formed inside the nozzle portion 20. The flow passage 201 opens at the distal end of the nozzle portion 20. The proximal end of the flow passage 201 communicates with the lumen 181. The syringe 16 is configured to discharge the medical fluid M from the distal end of the nozzle portion 20.

The lock portion 22 is provided at the distal end of the neck portion 26. The lock portion 22 includes a first cylindrical portion 34 disposed radially outward of the nozzle portion 20. The first cylindrical portion 34 and the nozzle portion 20 are radially spaced apart from each other. The radially outer side of the nozzle portion 20 is covered by the first cylindrical portion 34. The distal end of the first cylindrical portion 34 is disposed on the proximal side relative to the distal end of the nozzle portion 20. That is, the distal end of the nozzle portion 20 protrudes in the distal direction relative to the distal end of the first cylindrical portion 34.

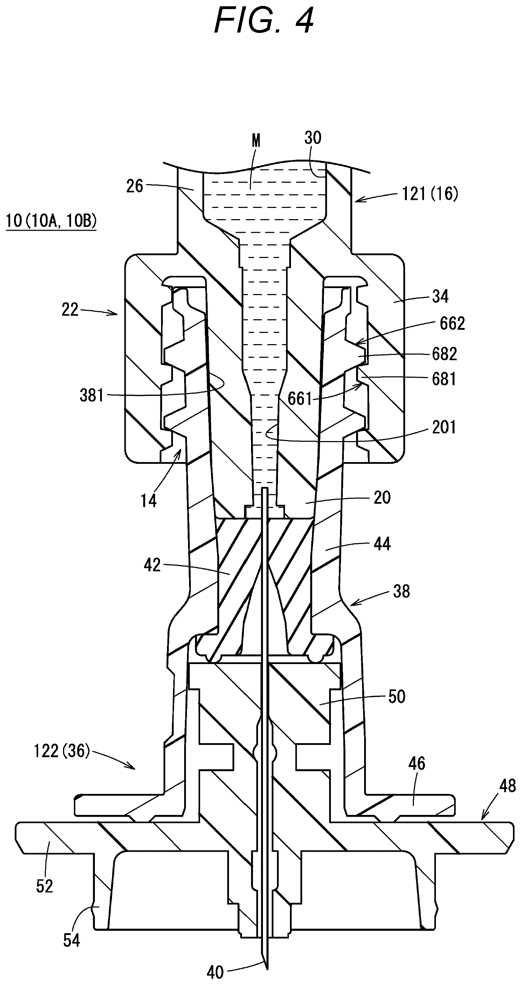

As illustrated in Fig. 2, the second medical device 122 is a puncture member 36. As illustrated in Fig. 4, the puncture member 36 includes a connector 38, a puncture needle 40 provided at the distal end of the connector 38, and an elastic body 42 provided in a hole portion 381 of the connector 38. The connector 38 is formed in a cylindrical shape having the hole portion 381. The connector 38 includes a second cylindrical portion 44 and a flange portion 46 provided at the distal end of the second cylindrical portion 44. An inner peripheral surface of the second cylindrical portion 44 is a taper extending in the distal direction. The inclination angle of the taper of the inner peripheral surface of the second cylindrical portion 44 is greater than the inclination angle of the Luer taper of the outer peripheral surface of the nozzle portion 20. Note that the inner peripheral surface of the second cylindrical portion 44 may be a Luer taper similarly to the outer peripheral surface of the nozzle portion 20. The hole portion 381 penetrates the second cylindrical portion 44 and the flange portion 46 in the axial direction of the connector 38. When the first medical device 121 and the second medical device 122 are connected, the nozzle portion 20 of the syringe 16 is inserted into the hole portion 381 from the proximal end of the second cylindrical portion 44 and accommodated therein. The flange portion 46 has a disk shape extending radially outward.

The puncture needle 40 is held by a needle hub 48. The needle hub 48 includes a needle holding portion 50 and a disk-shaped base portion 52 formed at the distal end of the needle holding portion 50. The needle holding portion 50 is inserted into the hole portion 381 from the distal end of the second cylindrical portion 44 and held therein. The center of the needle holding portion 50 holds the puncture needle 40. The proximal end of the puncture needle 40 protrudes in the proximal direction from the proximal end of the needle hub 48 and is disposed in the hole portion 381 of the connector 38. The distal end of the puncture needle 40 protrudes in the distal direction from the distal end of the base portion 52. The base portion 52 is disposed on the distal side of the flange portion 46 of the connector 38. The base portion 52 and the flange portion 46 are disposed in a state of being abutted against each other. The puncture needle 40 is disposed at the center of the base portion 52. The distal end of the puncture needle 40 is exposed to the outside relative to the base portion 52. The distal surface of the base portion 52 has an annular rib 54 protruding in the distal direction. The rib 54 is disposed radially outward and spaced apart, which is centered on the puncture needle 40. The rib 54 protrudes in the distal direction from the distal surface of the base portion 52. The distal end of the puncture needle 40 protrudes further in the distal direction than the distal end of the rib 54. As illustrated in Fig. 1, the outer peripheral surface of the base portion 52 has a protrusion 55 protruding radially outward.

As illustrated in Fig. 4, the elastic body 42 is formed of an elastic material such as rubber. The elastic body 42 is disposed in the hole portion 381 of the connector 38. When the syringe 16 (first medical device 121) and the puncture member 36 (second medical device 122) are connected, the elastic body 42 is disposed between the nozzle portion 20 and the needle holding portion 50 of the needle hub 48 in the hole portion 381. The proximal portion of the puncture needle 40 is inserted into the center of the elastic body 42. The elastic body 42 liquid-tightly seals a space between the hole portion 381 of the connector 38 and the needle holding portion 50.

When the first medical device 121 and the second medical device 122 are connected, the nozzle portion 20 of the syringe 16 is inserted into the hole portion 381 of the second cylindrical portion 44, and the elastic body 42 is pressed in the distal direction (axial direction) by the distal end of the nozzle portion 20. The elastic body 42 is compressed by being pressed in the distal direction by the nozzle portion 20, and is elastically deformed so as to expand radially outward. The elastic body 42 is in close contact with the inner peripheral surface of the hole portion 381.

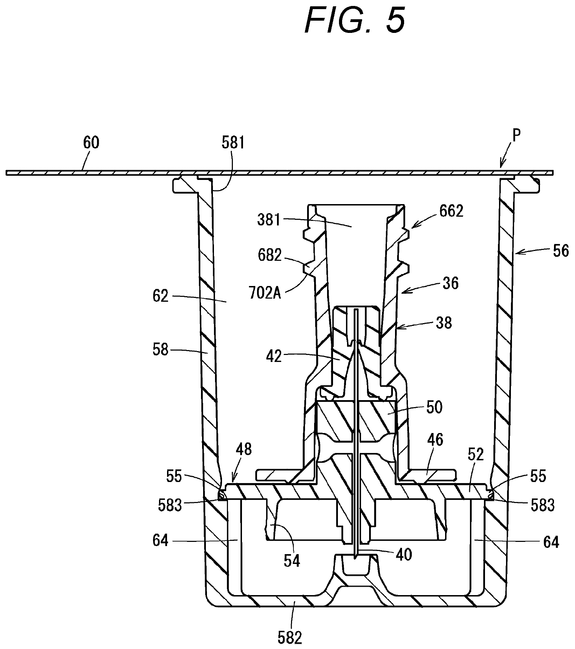

As illustrated in Fig. 5, the puncture member 36 is accommodated in a case 56 in a state before use. A medical apparatus 10A includes a puncture needle package P including the puncture needle 40 and the syringe 16 to which the puncture member 36 is connected. The puncture needle package P includes the puncture member 36 and the case 56 that is capable of accommodating the puncture member 36. The case 56 includes a case body 58 and a lid portion 60 provided to be openable at an opening 581 of the case body 58. The case body 58 has a cylindrical shape having the opening 581 at one end. The case body 58 includes an accommodation space 62 that accommodates the puncture member 36. The case body 58 has the opening 581 formed at one end and a bottom portion 582 formed at the other end opposite to the one end. When the puncture member 36 is accommodated in the accommodation space 62 of the case body 58, the base portion 52 of the needle hub 48 is disposed on the bottom portion 582 side, and the proximal end of the connector 38 is disposed on the opening 581 side.

The case body 58 includes a plurality of holding portions 64 protruding radially inward from the inner peripheral surface of the case body 58. The base portion 52 of the puncture member 36 is placed and held at the proximal ends of the plurality of holding portions 64. At this time, the distal end of the puncture needle 40 is disposed spaced apart from the bottom portion 582 of the case body 58. In the accommodation space 62, the puncture member 36 is accommodated in a state where relative rotation with respect to the case body 58 is restricted. The inner peripheral surface of the case body 58 has a recess 583 recessed radially outward. The protrusion 55 of the needle hub 48 is engaged with the recess 583. When the protrusion 55 is engaged with the recess 583, the rotation of the puncture member 36 with respect to the case 56 is prevented. Note that the rotation prevention of the needle hub 48 is not limited to a configuration in which the protrusion 55 of the needle hub 48 is engaged with the recess 583 of the case 56. For example, the recess 583 may not be provided on the inner peripheral surface of the case 56, and the protrusion 55 of the needle hub 48 may be lightly press-fitted into the inner peripheral surface of the case 56. The distal end of the protrusion 55 comes into contact with the inner peripheral surface of the case 56, and the rotation of the puncture member 36 is prevented by a frictional force therebetween. In this case, when a rotational force of a predetermined torque or more is applied to the puncture member 36, the rotational force exceeds the frictional force between the inner peripheral surface of the case 56 and the protrusion 55, such that the puncture member 36 rotates relative to the case 56.

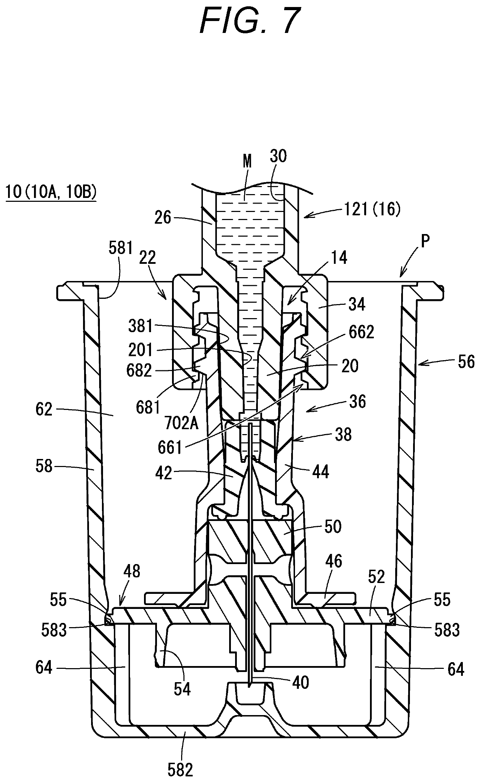

The lid portion 60 is formed of a resin material such as polyethylene resin. The lid portion 60 has a thin sheet shape having flexibility. The lid portion 60 is, for example, fused to the other end of the case body 58. The opening 581 is closed by the lid portion 60. The accommodation space 62 of the case body 58 is sealed by covering the opening 581 of the case body 58 with the lid portion 60. When connecting the puncture member 36 to the syringe 16, a user peels off the lid portion 60 from the case body 58 to open the opening 581 (see Fig. 7). At this time, the proximal end of the connector 38 of the puncture member 36 is accommodated so as to face the opening 581.

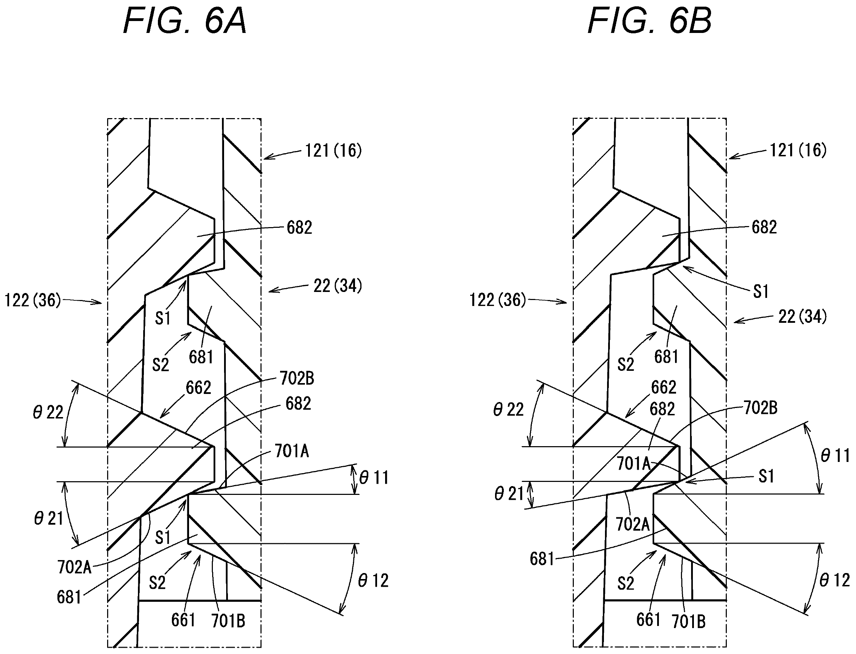

As illustrated in Fig. 4, the connection structure 14 includes a first threaded portion 661 provided in the first medical device 121 and a second threaded portion 662 provided on the second medical device 122 and screwed to the first threaded portion 661. The first threaded portion 661 is a tapered thread having a first thread ridge 681. The first threaded portion 661 is provided on the inner peripheral surface of the first cylindrical portion 34 of the lock portion 22 of the syringe 16. As illustrated in Fig. 6A, the first thread ridge 681 has a first engagement surface 701A and a first non-engagement surface 701B. The first engagement surface 701A is inclined at a first angle θ11 with respect to the radial direction orthogonal to the axis of the syringe 16. The first angle θ11 is, for example, in a range of 1° to 24°. The first angle θ11 is more preferably, for example, in a range of 5° to 20°. Alternatively, the first angle θ11 is, for example, 10° or 24°. In the axial direction of the first medical device 121, the first non-engagement surface 701B is disposed on the side opposite to the first engagement surface 701A at each first thread ridge 681.

As illustrated in Fig. 4, the second threaded portion 662 is a tapered thread having a second thread ridge 682. The second threaded portion 662 is provided in the connector 38 of the puncture member 36. The second threaded portion 662 is provided on the outer peripheral surface of the second cylindrical portion 44 in the connector 38. As illustrated in Fig. 6A, the second thread ridge 682 has a second engagement surface 702A and a second non-engagement surface 702B. The second engagement surface 702A is inclined at a second angle θ21 with respect to the radial direction orthogonal to the axis of the puncture member 36. The second angle θ21 is, for example, 25°. In the axial direction of the puncture member 36, the second non-engagement surface 702B is disposed on the side opposite to the second engagement surface 702A at each second thread ridge 682.

When the first threaded portion 661 and the second threaded portion 662 are screwed together, the first engagement surface 701A of the first threaded portion 661 and the second engagement surface 702A of the second threaded portion 662 come into contact with each other at a first portion S1 in the connection direction of the first medical device 121 and the second medical device 122. When the first threaded portion 661 and the second threaded portion 662 are screwed together, in the connection direction of the first medical device 121 and the second medical device 122, the first non-engagement surface 701B of the first thread ridge 681 of the first threaded portion 661 and the second non-engagement surface 702B of the second thread ridge 682 of the second threaded portion 662 are disposed at a second portion S2 provided on the side opposite to the first portion S1. At the second portion S2, the first non-engagement surface 701B and the second non-engagement surface 702B are not in contact with each other. The first portion S1 is a portion where the first engagement surface 701A and the second engagement surface 702A are in contact with each other in the connection direction (in Fig. 4, a vertical direction) of the first medical device 121 and the second medical device 122. At the first portion S1, the first angle θ11 of the first engagement surface 701A of the first thread ridge 681 is different from the second angle θ21 of the second engagement surface 702A of the second thread ridge 682.

Specifically, at the first portion S1, the first angle θ11 of the first engagement surface 701A of the first threaded portion 661 is smaller than the second angle θ21 of the second engagement surface 702A of the second threaded portion 662 (θ11 < θ21). An angle θ12 (hereinafter, referred to as a third angle θ12) of the first non-engagement surface 701B of the first thread ridge 681 at the second portion S2 is greater than the first angle θ11 of the first engagement surface 701A of the first thread ridge 681 at the first portion S1. Note that the third angle θ12 of the first non-engagement surface 701B of the first thread ridge 681 at the second portion S2 and the first angle θ11 of the first engagement surface 701A of the first thread ridge 681 at the first portion S1 may be the same.

For example, the difference between the first angle θ11 of the first engagement surface 701A of the first threaded portion 661 and the second angle θ21 of the second engagement surface 702A of the second threaded portion 662 is less than 25° (θ21 - θ11 < 25°). Note that a difference between the first angle θ11 of the first engagement surface 701A and the second angle θ21 of the second engagement surface 702A is not particularly limited as long as it is less than 25°. For example, the upper limit of the difference between the first angle θ11 of the first engagement surface 701A and the second angle θ21 of the second engagement surface 702A may be 24°, 22°, 20°, 18°, or 15°. The lower limit of the difference between the first angle θ11 of the first engagement surface 701A and the second angle θ21 of the second engagement surface 702A is not particularly limited as long as it is 0° or more. For example, the lower limit of the difference between the first angle θ11 of the first engagement surface 701A and the second angle θ21 of the second engagement surface 702A may be 2°, 5°, 8°, or 10°.

Note that, at the first portion S1, it is not limited to a case where the first angle θ11 of the first engagement surface 701A of the first threaded portion 661 is smaller than the second angle θ21 of the second engagement surface 702A of the second threaded portion 662. As illustrated in Fig. 6B, for example, at the first portion S1, the second angle θ21 of the second engagement surface 702A of the second threaded portion 662 may be smaller than the first angle θ11 of the first engagement surface 701A of the first threaded portion 661 (θ21 < θ11). That is, the first angle θ11 of the first engagement surface 701A and the second angle θ21 of the second engagement surface 702A at the first portion S1 are only required to be different from each other. In this case, an angle θ22 (hereinafter, referred to as a fourth angle θ22) of the second non-engagement surface 702B of the second thread ridge 682 at the second portion S2 is greater than the second angle θ21 of the second engagement surface 702A of the second thread ridge 682 at the first portion S1. Note that the fourth angle θ22 of the second non-engagement surface 702B at the second portion S2 and the second angle θ21 of the second engagement surface 702A at the first portion S1 may be the same.

As illustrated in Fig. 1, the syringe 16, which is the first medical device 121, and the puncture member 36, which is the second medical device 122, are connected to each other by the connection structure 14, thereby constituting a syringe assembly 10B, which is the medical device 10. Note that it is not limited to a case where the syringe 16, which is the first medical device 121, and the puncture member 36, which is the second medical device 122, are connected by the connection structure 14. For example, the first medical device other than the syringe 16 and the second medical device other than the puncture member 36 may be connected to each other by the connection structure 14 to constitute the medical device 10 other than the syringe assembly 10B.

Next, a case where the syringe assembly 10B as the medical device 10 is assembled by connecting the first medical device 121 and the second medical device 122 will be described.

As illustrated in Fig. 7, the user peels off the lid portion 60 of the puncture needle package P to open the opening 581 of the case body 58.

The distal portion of the syringe 16 is inserted into the accommodation space 62 of the case body 58, and the nozzle portion 20 is inserted into the connector 38. In a state where the case body 58 is fixed, the syringe 16 is moved in the distal direction while being rotated relative to the case body 58. The first threaded portion 661 of the first cylindrical portion 34 and the second threaded portion 662 of the second cylindrical portion 44 are screwed together. At this time, since the puncture member 36 is prevented from rotating relative to the case body 58, the puncture member 36 does not rotate in the accommodation space 62.

As illustrated in Fig. 6A, in the connection direction of the syringe 16 and the puncture member 36, the first engagement surface 701A of the first threaded portion 661 and the second engagement surface 702A of the second threaded portion 662 come into contact with each other at the first portion S1. When the syringe 16 is moved in the distal direction while being further rotated relative to the case body 58, the elastic body 42 is pressed in the distal direction by the distal end of the nozzle portion 20 as illustrated in Fig. 4. The proximal end of the puncture needle 40 is inserted into the flow passage 201 of the nozzle portion 20.

Thus, the first threaded portion 661 of the syringe 16 and the second threaded portion 662 of the puncture member 36 are screwed together, whereby the first medical device 121 (syringe 16) and the second medical device 122 (puncture member 36) are connected to each other.

After the medical device 10 in which the puncture member 36 is connected to the distal portion of the syringe 16 is assembled by the connection structure 14, the puncture member 36 connected to the syringe 16 is removed from the case 56 of the puncture needle package P. Thus, the assembly of the syringe assembly 10B, which is the medical device 10, is completed.

When the first engagement surface 701A of the first threaded portion 661 and the second engagement surface 702A of the second threaded portion 662 come into contact with each other at the first portion S1, the first angle θ11 of the first engagement surface 701A is smaller than the second angle θ21 of the second engagement surface 702A. Therefore, when assembling the syringe assembly 10B, the first engagement surface 701A and the second engagement surface 702A do not come into surface contact with each other, and the corner of the first thread ridge 681 of the first threaded portion 661 comes into contact with a part of the second engagement surface 702A. Thus, the corner of the top portion of the first thread ridge 681 bites into the second engagement surface 702A, whereby the first medical device 121 (syringe 16) and the second medical device 122 (puncture member 36) are firmly connected by the connection structure 14. In a case where the user rotates the syringe 16 more than necessary and a predetermined torque or more is applied to the puncture member 36 via the syringe 16, the protrusion 55 of the puncture member 36 is detached from the recess 583 of the case 56 in a state where the connection between the syringe 16 and the puncture member 36 is maintained. Thus, the puncture member 36 rotates relative to the case body 58 in a state where the connection between the syringe 16 and the puncture member 36 is maintained. That is, before the screw engagement between the first threaded portion 661 and second threaded portion 662 is released, the fixed state of the puncture member 36 with respect to the case body 58 is released, and the puncture member 36 rotates idly.

Next, a test performed to confirm the relationship between the torque applied at the time of connection and the first thread ridge 681 of the first threaded portion 661 in a case where the first medical device 121 and the second medical device 122 are connected by the connection structure 14 will be described.

### [Test Method]

First, the syringe 16 having the first threaded portion 661 is fixed to a digital torque gauge (Handy Type Torque Gauge HTG2-P Series, Model No. HTG2-5N-P, manufactured by IMADA Co., Ltd.), and then the puncture member 36 having the second threaded portion 662 is fixed inside the case body 58 with an adhesive or the like. Thus, the idle rotation of the puncture member 36 in the case body 58 is prevented. The case body 58 is set in a fixing block (not illustrated).

The first threaded portion 661 of the syringe 16 fixed to the digital torque gauge and the second threaded portion 662 of the puncture member 36 are screwed together to connect the syringe 16 and the puncture member 36 to each other, and then the puncture member 36 is rotated relative to the syringe 16 via the fixing block. The puncture member 36 is rotated until a screw engagement portion between the first threaded portion 661 and the second threaded portion 662 is broken, or until the first threaded portion 661 and the second threaded portion 662 move relative to each other in the radial direction, and the threaded engagement between the first threaded portion 661 and the second threaded portion 662 is released to cause idle rotation, and the maximum torque value thereof is measured by the digital torque gauge. The above-described test is performed a plurality of times using syringes 16 having different first angles θ11 of the first threaded portion 661.

### [Results]

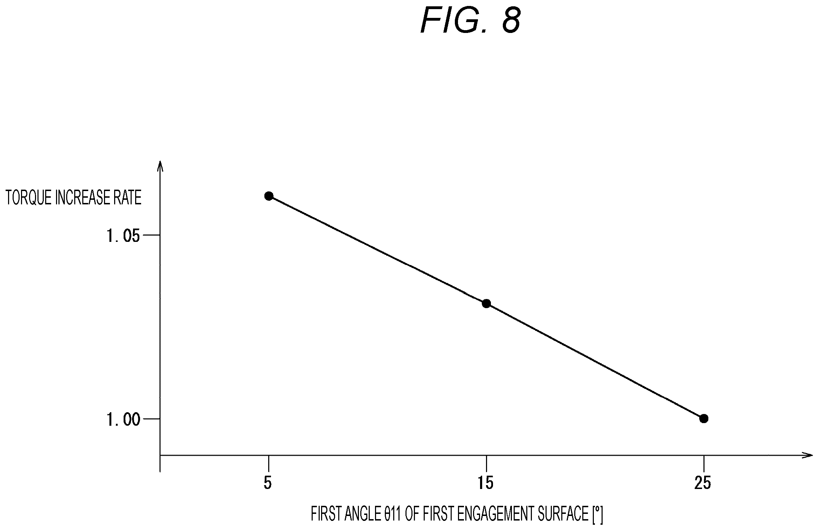

As understood from the characteristic diagram of Fig. 8 illustrating the relationship between the torque applied when connecting the syringe 16 and the puncture member 36 and the first angle θ11 of the first engagement surface 701A of the first thread ridge 681 of the first threaded portion 661, when the torque when the first angle θ11 of the first engagement surface 701A is 25° is 1.00, the torque increase rate is 1.03 when the first angle θ11 is 15° as compared with the case where the first angle θ11 is 25°, and the torque increase rate is 1.06 when the first angle θ11 is 5° as compared with the case where the first angle θ11 is 25°. The torque increase rate is a rate of increase in torque relative to the torque when the first angle θ11 of the first engagement surface 701A is 25°. As described above, it is recognized that as the first angle θ11 of the first engagement surface 701A of the first threaded portion 661 in the syringe 16 is decreased, the torque applicable at the time of the connection between the syringe 16 and the puncture member 36 increases. That is, it is recognized that by decreasing the first angle θ11 of the first engagement surface 701A, the syringe 16 having the first threaded portion 661 and the puncture member 36 having the second threaded portion 662 can be more firmly connected to each other.

Next, a case of administering the medical fluid M to a patient 300 using the syringe assembly 10B will be described.

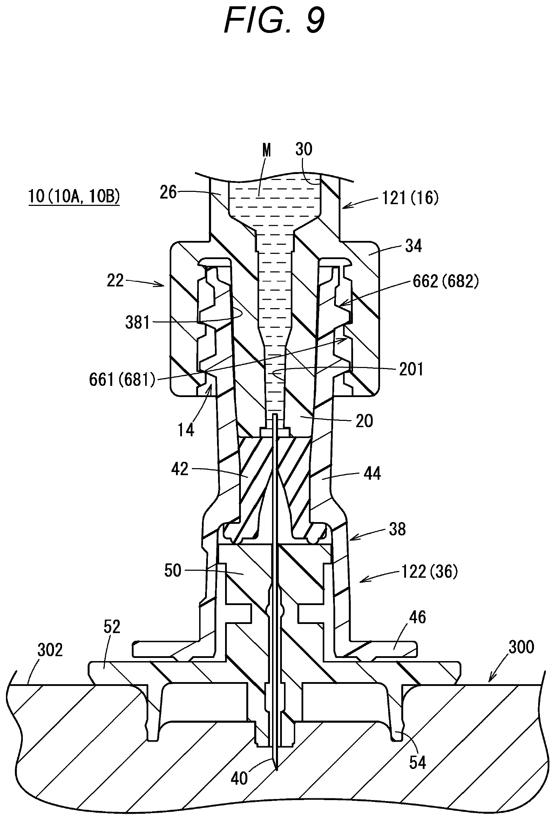

As illustrated in Fig. 9, by pressing the base portion 52 of the puncture member 36 against the skin 302 of the patient 300, a part of the skin 302 is pressed into the inside of the rib 54 by the rib 54. The skin 302 bulges toward the base portion 52 inside the rib 54, and the needle tip of the puncture needle 40 punctures the center of the bulged skin 302. After confirming the puncture of the skin 302 using the puncture needle 40, the user pushes the proximal end of the plunger 32 in the distal direction, whereby the medical fluid M is compressed in the distal direction by the gasket 28. The medical fluid M in the drug chamber 30 is administered subcutaneously from the distal end through the flow passage 201 of the nozzle portion 20 and the lumen of the puncture needle 40.

The present embodiment provides the following effects.

As illustrated in Fig. 4, the medical device 10 includes the first medical device 121 having the first threaded portion 661 and the second medical device 122 having the second threaded portion 662 screwed to the first threaded portion 661, and has the first portion S1 where the first engagement surface 701A of the first thread ridge 681 and the second engagement surface 702A of the second thread ridge 682 come into contact with each other when the first threaded portion 661 and the second threaded portion 662 are screwed together. At the first portion S1, the first angle θ11 of the first engagement surface 701A of the first thread ridge 681 of the first threaded portion 661 is different from the second angle θ21 of the second engagement surface 702A of the second thread ridge 682 of the second threaded portion 662.

Thus, when the first threaded portion 661 and the second threaded portion 662 are screwed together, the first engagement surface 701A and the second engagement surface 702A, which have mutually different angles (the first angle θ11 and the second angle θ21) at the first portion S1, do not come into surface contact with each other. Therefore, when the first threaded portion 661 and the second threaded portion 662 are screwed together, the relative movement of the first threaded portion 661 and the second threaded portion 662 in the radial direction along the first engagement surface 701A and the second engagement surface 702A, which causes them to ride over each other, is effectively suppressed. Therefore, the first threaded portion 661 and the second threaded portion 662 can be firmly connected.

As illustrated in Fig. 4, the first medical device 121 is the syringe 16 including the lock portion 22 surrounding the nozzle portion 20, and the second medical device 122 is the puncture member 36 including the connector 38 having the hole portion 381 capable of accommodating the nozzle portion 20 and the puncture needle 40 provided at the distal end of the connector 38. The lock portion 22 includes the first cylindrical portion 34 having the first threaded portion 661 on the inner peripheral surface, and the connector 38 includes the second cylindrical portion 44 having the second threaded portion 662 on the outer peripheral surface. Thus, by firmly connecting the first threaded portion 661 of the first cylindrical portion 34 and the second threaded portion 662 of the second cylindrical portion 44, it is possible to effectively suppress leakage of the medical fluid M when the medical device 10, in which the syringe 16 and the puncture member 36 are connected, administers the medical fluid.

As illustrated in Fig. 6A, the first angle θ11 of the first engagement surface 701A of the first thread ridge 681 of the first threaded portion 661 is smaller than the second angle θ21 of the second engagement surface 702A of the second thread ridge 682 of the second threaded portion 662. Thus, when the first threaded portion 661 and the second threaded portion 662 are screwed together, the first thread ridge 681 of the first threaded portion 661 bites into the second engagement surface 702A of the second thread ridge 682 of the second threaded portion 662, whereby the first medical device 121 and the second medical device 122 can be firmly connected.

As illustrated in Fig. 6B, the second angle θ21 of the second engagement surface 702A of the second thread ridge 682 of the second threaded portion 662 is smaller than the first angle θ11 of the first engagement surface 701A of the first thread ridge 681 of the first threaded portion 661. Thus, when the first threaded portion 661 and the second threaded portion 662 are screwed together, the second thread ridge 682 of the second threaded portion 662 bites into the first engagement surface 701A of the first threaded portion 661, whereby the first medical device 121 and the second medical device 122 can be firmly connected.

As illustrated in Fig. 4, the puncture member 36 includes the elastic body 42 that is disposed in the hole portion 381 of the connector 38 and is pressed and compressed by the distal end of the nozzle portion 20 when the syringe 16 is connected. Thus, when the syringe 16 and the puncture member 36 are connected by the connection structure 14, the elastic body 42 is compressed by the distal end of the nozzle portion 20, whereby it is possible to effectively suppress leakage of the medical fluid M through a space between the distal end of the syringe 16 and the hole portion 381 of the puncture member 36 by the elastic body 42. Even when the syringe 16 and the puncture member 36 are biased in the direction of being separated from each other by the resilient force, the first thread ridge 681 and the second thread ridge 682 are effectively prevented from riding over each other in the radial direction.

As illustrated in Fig. 6A, with the second portion S2 provided on the side opposite to the first portion S1 in the connection direction of the first medical device 121 and the second medical device 122, the third angle θ12 of the first non-engagement surface 701B of the first thread ridge 681 at the second portion S2 is greater than the first angle θ11 of the first engagement surface 701A of the first thread ridge 681 at the first portion S1. Thus, since the third angle θ12 of the first non-engagement surface 701B of the first thread ridge 681 at the second portion S2 can be set to be the same as the second angle θ21 of the second engagement surface 702A of the second thread ridge 682, the first threaded portion 661 can be easily formed at low cost as compared with a configuration in which both the first angle θ11 of the first engagement surface 701A and the third angle θ12 of the first non-engagement surface 701B of the first thread ridge 681 at the first and second portions S1 and S2 are smaller than the second angle θ21 of the second engagement surface 702A of the second thread ridge 682.

As illustrated in Fig. 6B, when the first threaded portion 661 and the second threaded portion 662 are screwed together, with the second portion S2 provided on the side opposite to the first portion S1 in the connection direction of the first medical device 121 and the second medical device 122, the fourth angle θ22 of the second non-engagement surface 702B of the second thread ridge 682 at the second portion S2 is greater than the second angle θ21 of the second engagement surface 702A of the second thread ridge 682 at the first portion S1. Thus, the fourth angle θ22 of the second non-engagement surface 702B of the second thread ridge 682 at the second portion S2 can be set to be the same as the first angle θ11 of the first engagement surface 701A of the first thread ridge 681. Therefore, as compared with a configuration in which both the second angle θ21 of the second engagement surface 702A and the fourth angle θ22 of the second non-engagement surface 702B of the second thread ridge 682 at the first and second portions S1 and S2 are smaller than the first angle θ11 of the first engagement surface 701A of the first thread ridge 681, the second threaded portion 662 can be easily formed at low cost.

As illustrated in Fig. 6A, the difference between the first angle θ11 of the first engagement surface 701A of the first threaded portion 661 and the second angle θ21 of the second engagement surface 702A of the second threaded portion 662 is less than 25°. Thus, the first medical device 121 and the second medical device 122 can be more firmly connected.

As illustrated in Fig. 1, the syringe assembly 10B includes the syringe 16 and the puncture member 36. Thus, the syringe 16 and the puncture member 36, which constitute the syringe assembly 10B, can be firmly connected.

As illustrated in Fig. 7, the syringe 16, the puncture member 36, and the case 56 in which the puncture member 36 is accommodated and held are provided. When the puncture member 36 and the syringe 16 are connected by the first threaded portion 661 and the second threaded portion 662 in a state where the puncture member 36 is held inside the case 56, the puncture member 36 rotates relative to the case 56 when a predetermined torque or more acts on the puncture member 36 as the first threaded portion 661 and the second threaded portion 662 are screwed together.

Thus, even in a case where a predetermined torque or more to the extent that the puncture member 36 and the case 56 rotate relative to each other acts on the puncture member 36, the screw engagement between the first threaded portion 661 and the second threaded portion 662 is firm, such that the screw engagement between the first threaded portion 661 and the second threaded portion 662 is effectively maintained.

Note that the present invention is not limited to the above disclosure and can take various configurations without departing from the gist of the present invention.

## Claims

1. A connection structure of a medical device, the connection structure connecting a first medical device having a first threaded portion and a second medical device having a second threaded portion screwed to the first threaded portion,
wherein the first threaded portion of the first medical device is a tapered thread having a first thread ridge,
the second threaded portion of the second medical device is a tapered thread having a second thread ridge that comes into contact with the first thread ridge of the first threaded portion,
when the first threaded portion and the second threaded portion are screwed together, a first portion is provided where a first engagement surface of the first thread ridge and a second engagement surface of the second thread ridge come into contact with each other,
the first engagement surface is inclined at a first angle with respect to a direction orthogonal to an axis of the first medical device, and the second engagement surface is inclined at a second angle with respect to a direction orthogonal to an axis of the second medical device, and
at the first portion, the first angle of the first engagement surface of the first thread ridge is different from the second angle of the second engagement surface of the second thread ridge.

2. The connection structure of a medical device according to claim 1,
wherein the first medical device is a syringe including a main body portion capable of being filled with a medical fluid, a nozzle portion provided at a distal end of the main body portion, and a lock portion surrounding the nozzle portion,
the second medical device is a puncture member including a connector having a hole portion capable of accommodating the nozzle portion and a puncture needle provided at a distal end of the connector, and
the lock portion includes a first cylindrical portion having the first threaded portion on an inner peripheral surface, and the connector includes a second cylindrical portion having the second threaded portion on an outer peripheral surface.

3. The connection structure of a medical device according to claim 2,
wherein the first angle of the first engagement surface of the first threaded portion is smaller than the second angle of the second engagement surface of the second threaded portion.

4. The connection structure of a medical device according to claim 2,
wherein the second angle of the second engagement surface of the second threaded portion is smaller than the first angle of the first engagement surface of the first threaded portion.

5. The connection structure of a medical device according to claim 2,
wherein the puncture member includes an elastic body disposed in the hole portion of the connector, and pressed and compressed by a distal end of the nozzle portion when the syringe is connected.

6. The connection structure of a medical device according to claim 3,
wherein when the first threaded portion and the second threaded portion are screwed together, in a connection direction of the first medical device and the second medical device, a second portion is provided on a side opposite to the first portion, and
an angle of a first non-engagement surface of the first thread ridge at the second portion is greater than the first angle of the first engagement surface of the first thread ridge at the first portion.

7. The connection structure of a medical device according to claim 4,
wherein when the first threaded portion and the second threaded portion are screwed together, in a connection direction of the first medical device and the second medical device, a second portion is provided on a side opposite to the first portion, and
an angle of a second non-engagement surface of the second thread ridge at the second portion is greater than the second angle of the second engagement surface of the second thread ridge at the first portion.

8. The connection structure of a medical device according to claim 1,
wherein a difference between the first angle of the first engagement surface of the first threaded portion and the second angle of the second engagement surface of the second threaded portion is less than 25°.

9. A syringe assembly that has the connection structure of a medical device according to claim 2, the syringe assembly comprising the syringe and the puncture member.

10. A medical apparatus including the syringe assembly according to claim 9, the medical apparatus comprising: the syringe; the puncture member; and a case in which the puncture member is accommodated and held,
wherein in a state where the puncture member is held inside the case, the puncture member and the syringe are capable of being connected by the first threaded portion and the second threaded portion, and
when the syringe and the puncture member are connected, the puncture member rotates relative to the case when a predetermined torque or more acts on the puncture member as the first threaded portion and the second threaded portion are screwed together.
